# EUROPEAN PATENT SPECIFICATION

(11) **EP 0 803 235 B1**
(45) Date of publication and mention of the grant of the patent: **25.09.2002**
(21) Application number: 96302875.8
(22) Date of filing: 25.04.1996
(51) Int. Cl.: A61F 5/44

(54) **A tap or valve**
Hahn oder Ventil
Robinet ou valve

(43) Date of publication of application: 29.10.1997
(73) Proprietor: BRISTOL-MYERS SQUIBB COMPANY, Princeton, NJ 08543-4000 (US)
(72) Inventor: Falconer, Malcolm Ian, Wandsworth Common, London SW18 3NQ (GB)
(74) Representative: Holmes, Miles Keeton

(56) References cited:
- GB-A- 2 283 077
- US-A- 3 400 866
- US-A- 4 211 348

## Description

This invention relates to a tap of valve and particularly although not exclusively to a tap for a urostomy pouch.

Various designs of taps or dispensing spigots have been proposed. See for example Fattori et al U.S. Patents Nos. 3,400,866 and 4,787,538, Welsh U.S. Patent No. 3,972,452, European Patent Application No. 95861A and Scholle U.S. Patent No. 4,211,348. These designs, in the Applicants' opinion, fall short in that they are lacking in simplicity. Most are completely unsuitable for employment in a urostomy pouch.

GB-A-2283077 in contrast discloses a plastics tap for controlling exit of liquid from a urostomy pouch. The tap consists of two parts, the first part having a flat flange surrounding an opening and being connected to a hollow generally tubular member. The member has a radially inwardly projecting rim surrounding the opening near to the flange, and the end of the member remote from the opening has a radially outwardly extending rim, there being a hole through the tubular member at one radial location. The second part has a collar-like first portion which snap-fits over the rim and has a hole in registry with the hole in the tubular member, and a tubular second portion which serves as the valve member. The second portion is located radially inwardly of the first portion and the tubular member, and has one open end. A radially outer sealing surface at the open end provides closure contact with the rim. The first and second parts are integrally joined by a deformable plastics annulus which is constructed to permit axial movement of the second portion, and its sealing surface, towards and away from the rim.

The present invention provides a tap for a liquid container, made from natural or synthetic rubber or a resilient plastics material, which comprises a base portion having an orifice which serves as a liquid inlet therein, a closure portion, and a tap exit, the closure portion being constructed to make a snap-on connection with a periphery of the base portion, the closure portion comprising a resiliently deformable zone and a non-deformable zone, the non-deformable zone having a pushable projecting portion extending away from the base portion and a plug extending towards the base portion, the deformable zone biasing the plug so as to obturate the orifice and close the liquid inlet, characterised in that the deformable zone is configured as a hinge such that, when the non-deformable zone is pushed, the deformable zone hinges so as so tilt the plug away from the inlet orifice by a hinge action, thereby permitting the flow of liquid to the tap exit.

In a preferred embodiment of the invention, the closure portion is a wall which is generally oval in shape as seen in front elevation and has an external thickened sloping portion upon which the plug is internally mounted. The sloping portion is located generally centrally of the closure portion. In the embodiment of the invention which consists of a urostomy pouch, the base portion including the liquid exit is constructed for attachment, e.g. by heat or R.F. welding, to one wall of the pouch, the pouch having an aperture in one of its walls, the aperture being in registry with the liquid inlet orifice.

In an advantageous embodiment of the invention, a night drainage adapter may be used with, and temporarily incorporated in, the tap described and illustrated herein. This adapter device comprises a tube extension of a particular shape which when inserted into the liquid exit means, holds the closure portion away from the liquid entry hole in the base portion. This permits intermittent night drainage to occur, with no manipulation to open the tap being necessary.

The invention will be better understood from the following description of an illustrative embodiment, in which like parts are represented by like reference numerals, and in which:-
Figure 1 is a front view of one example of tap according to the invention;
Figure 2 is a cross-section on the plane II-II in Figure 1 showing the tap in its open condition and diagrammatically illustrating part of a wall of a urostomy pouch;
Figure 3 is a view identical to Figure 2 but showing the tap in its normally closed condition;
Figure 4 is a cross-sectional view of a tap having a night drainage adapter in place; and
Figure 5 illustrates an optional refinement in the form of a peripheral groove on the drain tube, which allows attachment of a known type of tube connector (see UK Patent 2,092,690; EP 57057B; and UK Patent Application No. 9514011.7).

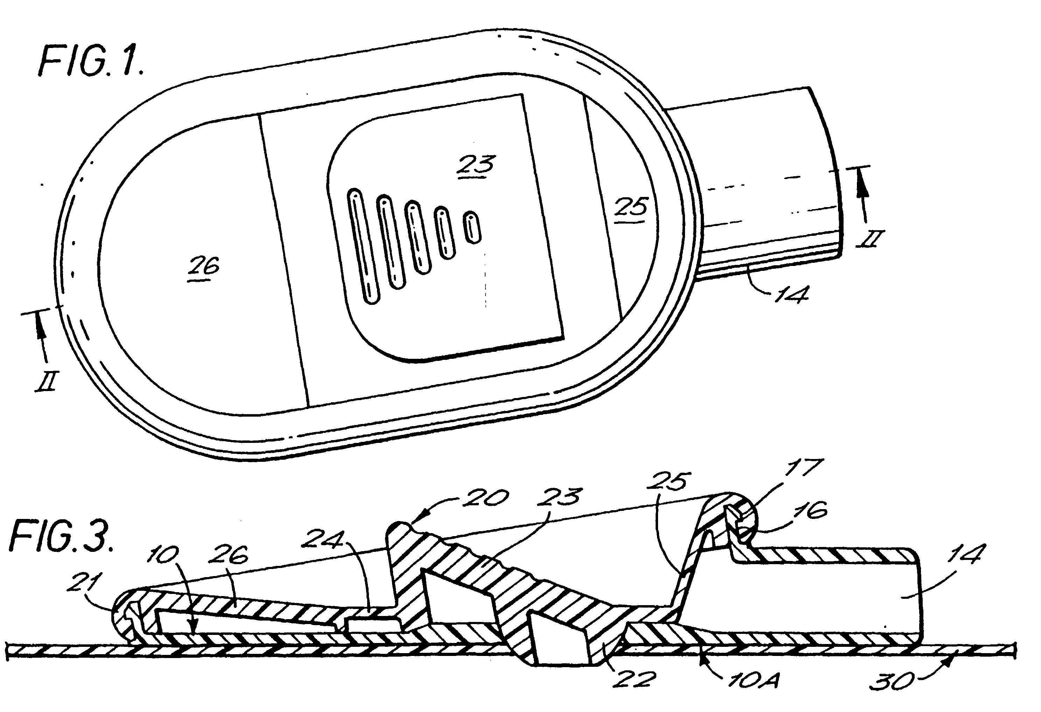

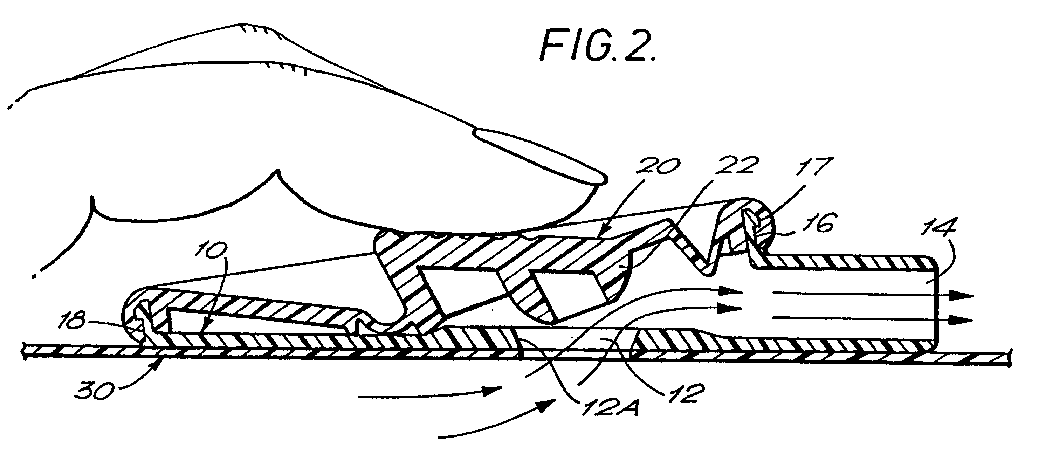

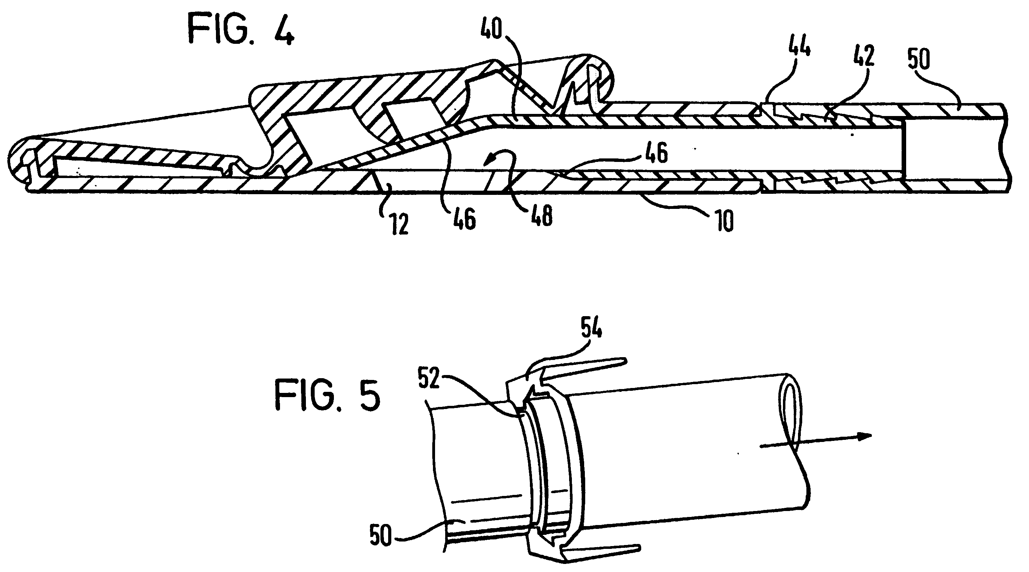

Referring to Figures 1-3, the illustrated tap comprises a base portion 10 and a closure portion 20. The wall of a urostomy pouch which is the currently preferred example of product in which the tap may be employed, is shown at 30 in Figures 2 and 3.

The base portion 10 comprises a central orifice 12 and embodies a liquid exit means 14. The base portion has a peripheral wall 16, and carried by this wall is an outwardly extending rim 17 provided to enable snap-on engagement between itself and a connection means 21 on the periphery of the closure portion 20. The base portion 10 has a flat surface 10A which may be plastics welded or adhesively secured or fastened in any other convenient way to the wall 30 of the liquid container.

The peripheral snap-on connection means 21 of the closure portion is designed to co-operate with the peripheral wall 16 of the base portion. The closure portion is made of a resilient deformable material, preferably a thermoplastics elastomer. The closure portion has a centrally located plug 22, which extends towards the base portion 10 and is shaped to make a complementary mating engagement with the wall 12A of the orifice 12. The closure portion has a peripheral slot 18, in which is received the wall 16.

The left-hand end as seen in Figure 2 or Figure 3 of the base and closure portions will be uppermost in use of the tap, with the liquid exit means 14 being lowermost.

The closure portion 20 is a plastics moulding, and has a thickened portion 23 which in the closed position of the tap is at an angle to the vertical and in the tap's open position occupies an approximately vertical position. This may be achieved by manipulating the closure portion 20 with a finger as illustrated in Figure 2. This manipulation is faciliated by the fact that the thickness of the closure portion is substantially reduced at the zones 24 and 25 and is reduced but to a lesser extent at the zone 26. The zones 24 and 25 readily deform to enable the closure portion to take up its open position as illustrated in Figure 2.

One desirable advantage of this design of tap is that its overall thickness is small. Hence, a pouch having this design of tap is unobtrusive when worn. Another advantage is that the closure portion 20 immediately reverts to the closed position as seen in Figure 3 when the finger pressure is removed from the portion 23 thereof. The complementary shaping of the plug 22 and the wall 12A, and the interfit between the parts 16, 17, 18, assist in achieving a durable and leakproof fit. The tap as illustrated in Figures 1-3 has no detachable parts which might become misplaced or lost.

A night drainage adapter 40 is illustrated in Figure 4 in position within the tap. The adapter 40 comprises a tapered tube having a ribbed end 42, a flange 44, a taper 46 at its upper end in use, and a hole 48 of a size slightly greater than the hole 12 in the base portion 10. The ribbed end 42 facilitates connection to a drain tube 50. As will be seen in Figure 4, the adapter 40 when inserted upwardly into the tap, forces the closure portion away from the base portion 10. The resilience of the closure portion holds the adaptor 40 into close engagement with the surface of the base portion. Liquid entering via hole 12 is then guided by adapter 40 directly to the drain tube 50.

Referring now to Figure 5, at the lower end of the drain tube 50 a peripheral groove 52 is provided, to receive hook members 54 of a known connector, provided to convey liquid, e.g. to a night drainage bag or reservoir.

## Claims

1. A tap for a liquid container, made from natural or synthetic rubber or a resilient plastics material, which comprises a base portion (10) having an orifice (12) which serves as a liquid inlet therein, a closure portion (20), and a tap exit (14), the closure portion (20) being constructed to make a snap-on connection with a periphery (17) of the base portion (10), the closure portion (20) comprising a resiliently deformable zone (24, 25) and a non-deformable zone (23), the non-deformable zone having a pushable projecting portion extending away from the base portion and a plug (22) extending towards the base portion, the deformable zone (24, 25) biasing the plug (22) so as to obturate the orifice (12) and close the liquid inlet, **characterised in that** the deformable zone (24, 25) is configured as a hinge such that, when the non-deformable zone (23) is pushed, the deformable zone (24, 25) hinges so as so tilt the plug (22) away from the inlet orifice (12) by a hinge action, thereby permitting the flow of liquid to the tap exit.

2. A tap according to claim 1, wherein the base portion (10) comprises a first surface and a second surface, the closure portion (20) being mounted on the first surface, the deformable zone (24, 25) biasing the plug (22) in a direction from the closure portion (20) towards the first surface, and wherein, when the non-deformable zone (23) is pushed, the plug (22) lifts away from the first surface.

3. A tap according to claim 1 or 2, wherein the non-deformable zone (23) of the closure portion (20) is thicker than the deformable zone (24,25).

4. A tap according to claim 1,2 or 3, wherein the non-deformable zone (23) has a sloping portion on which the plug (22) is carried.

5. A tap according to claim 4, wherein the sloping portion is located centrally of the closure portion (20).

6. A tap according to claim 1, 2, 3 or 4, wherein the tap exit (14) is embodied in the base portion (10).

7. A combination of a tap according to any preceding claim, and a urostomy pouch (30), the base portion (10) being attached to the exterior of the pouch with the orifice (12) in registry with a hole in the pouch wall.

8. A combination of a tap as defined in any of claims 1 to 6, and a night drainage adapter (40).

9. A combination according to claim 8, wherein the night drainage adapter (40) comprises a tube having an end portion insertable into the exit of the tap, so as to prevent obturation of the orifice (12) by the plug (22) of the closure portion (20).

10. A combination according to claim 9, wherein the end portion is tapered.

11. A combination according to claim 9, wherein the end portion of the tube is shaped so as to force, upon insertion of the tube into the exit of the tap, said closure portion (20) away from the base portion (10) and said plug (22) from the inlet orifice (12).

## Patentansprüche

1. Aus einem Natur- oder Synthesekautschuk oder einem elastischen Kunststoffmaterial bestehender Hahn für einen Flüssigkeitsbehälter, der einen Grundteil (10) mit einer Öffnung (12), die darin als Flüssigkeitseinlass dient, einen Schließteil (20) und einen Hahnauslass (14) hat, wobei der Schließteil (20) so konstruiert ist, dass er mit einer Peripherie (17) des Grundteils (10) eine Aufschnapp-Verbindung bildet, wobei der Schließteil (20) einen elastisch verformbaren Bereich (24, 25) und einen nichtverformbaren Bereich (23) aufweist, wobei der nichtverformbare Bereich einen schiebbaren vorspringenden Teil, der vom Grundteil weg verläuft, und ein Küken (22), das zum Grundteil hin verläuft, hat, wobei der verformbare Bereich (24, 25) das Küken (22) vorbetastet, um die Öffnung (12) zu verschließen und den Flüssigkeitseinlass zu schließen, **dadurch gekennzeichnet, dass** der verformbare Bereich (24, 25) als Drehgelenk konfiguriert ist, sodass sich beim Schieben des nichtverformbaren Bereiches (23) der verformbare Bereich (24, 25) so dreht, dass er das Küken (22) durch Drehen von der Einlassöffnung (12) weg kippt, wodurch die Flüssigkeit zum Hahnauslass fließen kann.

2. Hahn nach Anspruch 1, **dadurch gekennzeichnet, dass** der Grundteil (10) eine erste Fläche und eine zweite Fläche aufweist, wobei der Schließteil (20) an der ersten Fläche montiert ist und der verformbare Bereich (24, 25) das Küken (22) in einer Richtung vom Schließteil (20) hin zur ersten Fläche vorbelastet, und dass beim Schieben des nichtverformbaren Bereiches (23) sich das Küken (22) von der ersten Fläche weg hebt.

3. Hahn nach Anspruch 1 oder 2, **dadurch gekennzeichnet, dass** der nichtverformbare Bereich (23) des Schließteils (20) dicker als der verformbare Bereich (24, 25) ist.

4. Hahn nach Anspruch 1, 2 oder 3, **dadurch gekennzeichnet, dass** der nichtverformbare Bereich (23) einen geneigten Teil hat, an dem das Küken (22) gehalten wird.

5. Hahn nach Anspruch 4, **dadurch gekennzeichnet, dass** sich der geneigte Teil in der Mitte des Schließteils (20) befindet.

6. Hahn nach Anspruch 1, 2, 3 oder 4, **dadurch gekennzeichnet, dass** der Hahnauslass (14) im Grundteil (10) integriert ist.

7. Kombination aus einem Hahn nach einem vorhergehenden Anspruch und einem Urostomie-Beutel (30), wobei der Grundteil (10) an der Außenseite des Beutels befestigt ist, wobei die Öffnung (12) mit einem Loch in der Beutelwand übereinstimmt.

8. Kombination aus einem in einem der Ansprüche 1 bis 6 definierten Hahn und einem Nachtablaufadapter (40).

9. Kombination nach Anspruch 8, **dadurch gekennzeichnet, dass** der Nachtablaufadapter (40) einen Schlauch mit einem Endstück aufweist, das in den Hahnauslass einsteckbar ist, um das Verschließen der Öffnung (12) mit dem Küken (22) des Schließteils (20) zu vermeiden.

10. Kombination nach Anspruch 9, **dadurch gekennzeichnet, dass** das Endstück kegelförmig ist.

11. Kombination nach Anspruch 9, **dadurch gekennzeichnet, dass** das Endstück des Schlauches so gestaltet ist, dass es nach dem Einstecken des Schlauches in den Hahnauslass den Schließteil (20) vom Grundteil (10) und das Küken (22) von der Einlassöffnung (12) weg drückt.

## Revendications

1. Robinet destiné à un récipient de liquide, réalisé à partir de caoutchouc naturel ou synthétique ou d'une matière plastique élastique, qui comprend une partie de base (10) comportant un orifice (12) qui sert d'entrée de liquide dans celle-ci, une partie de fermeture (20), et une sortie de robinet (14), la partie de fermeture (20) étant conçue pour réaliser un raccordement par encliquetage avec une périphérie (17) de la partie de base 10, la partie de fermeture (20) comprenant une zone déformable de façon élastique (24, 25) et une zone non déformable (23), la zone non déformable comportant une partie saillante pouvant être poussée s'étendant à l'écart de la partie de base et un bouchon (22) s'étendant en direction de la partie de base, la zone déformable (24, 25) sollicitant le bouchon (22) de façon à obturer l'orifice (12) et fermer l'entrée de liquide, **caractérisé en ce que** la zone déformable (24, 25) est configurée sous forme d'une charnière de sorte que, lorsque la zone non déformable (23) est poussée, la zone déformable (24, 25) pivote de façon à basculer le bouchon (22) à l'écart de l'orifice d'entrée (12) par une action de charnière, en permettant ainsi l'écoulement de liquide vers la sortie du robinet.

2. Robinet selon la revendication 1, dans lequel la partie de base (10) comprend une première surface et une seconde surface, la partie de fermeture (20) étant montée sur la première surface, la zone déformable (24, 25) sollicitant le bouchon (22) dans une direction depuis la partie de fermeture (20) vers la première surface, et dans lequel, lorsque la zone non déformable (23) est poussée, le bouchon (22) se soulève à l'écart de la première surface.

3. Robinet selon la revendication 1 ou 2, dans lequel la zone non déformable (23) de la partie de fermeture (20) est plus épaisse que la zone déformable (24, 25).

4. Robinet selon la revendication 1, 2 ou 3, dans lequel la zone non déformable (23) comporte une partie inclinée sur laquelle le bouchon (22) est supporté.

5. Robinet selon la revendication 4, dans lequel la partie inclinée est située au centre de la partie de fermeture (20).

6. Robinet selon la revendication 1, 2, 3 ou 4, dans lequel la sortie du robinet (14) est incorporée dans la partie de base (10) .

7. Combinaison d'un robinet selon l'une quelconque des revendications précédentes, et d'une poche d'urostomie (30), la partie de base (10) étant fixée à l'extérieur de la poche l'orifice (12) se trouvant en alignement avec un trou dans la paroi de la poche.

8. Combinaison d'un robinet selon l'une quelconque des revendications 1 à 6 et d'un adaptateur d'évacuation nocturne (40) .

9. Combinaison selon la revendication 8, dans laquelle l'adaptateur d'évacuation nocturne (40) comprend un tube ayant une partie d'extrémité pouvant être insérée dans la sortie du robinet, de façon à empêcher l'obturation de l'orifice (12) par le bouchon (22) de la partie de fermeture (20).

10. Combinaison selon la revendication 9, dans laquelle la partie d'extrémité est conique.

11. Combinaison selon la revendication 9, dans laquelle la partie d'extrémité du tube est profilée de façon à, lors de l'insertion du tube dans la sortie du robinet, éloigner de force ladite partie de fermeture (20) de la partie de base (10) et ledit bouchon (20) de l'orifice d'entrée (12).
